# EUROPEAN PATENT APPLICATION

(11) **EP 4 835 904 A1**
(43) Date of publication of application: **04.11.2026**
(21) Application number: 24913284.6
(22) Date of filing: 20.09.2024
(51) Int. Cl.: G16H 50/20, G16H 30/40, G06T 7/00, G06T 7/10, A61B 6/03, G06T 5/20, G06T 11/00

(54) **ARTIFICIAL INTELLIGENCE-BASED APPARATUS AND METHOD FOR PREDICTING LARGE VASCULAR OCCLUSION**

(30) Priority: 29.12.2023 KR 20230197141
(71) Applicant: JLK Inc., Cheongju-si, Chungcheongbuk-do 28118 (KR); Dongguk University Industry-Academic Cooperation Foundation, Seoul 04620 (KR)
(72) Inventor: KANG, Shin Uk, Seoul 06276 (KR); LEE, Myung Jae, Seoul 06090 (KR); KIM, Dong Min, Seoul 06107 (KR); RYU, Wi Sun, Incheon 22002 (KR); KIM, Pyeong Eun, Seoul 06221 (KR); KIM, Dong Eog, Seoul 06509 (KR)
(74) Representative: Zacco Sweden AB
(86) International application number: PCT/KR2024/014218
(87) International publication number: WO 2025/143443

(57) **Abstract**

A method for an artificial intelligence-based predicting a large vessel occlusion. The method comprising inputting a brain scan image obtained by imaging equipment, calculating a characteristic value for each brain region by masking a brain region mask onto the brain scan image, and calculating a brain lesion characteristic value based on whether a brain lesion region is segmented and outputting a confidence score for the large vessel occlusion by inputting the characteristic value for each brain region and the brain lesion characteristic value to a prediction artificial intelligence.

## Description

### [Technical Field]

The present disclosure relates to an artificial intelligence (AI)-based apparatus and method of predicting a large vessel occlusion, and more particularly, to an AI-based apparatus and method of predicting a large vessel occlusion, which extract various characteristics from an image captured without a contrast agent and predict a large vessel occlusion based on the various characteristics.

### [Background Art]

A conventional AI model that predicts a large vessel occlusion from non-contrast computed tomography (NCCT), which is a CT image captured without a contrast agent, has a tendency to be dependent on a clot (clot sign) and thus has a limitation in achieving the consistent performance across different races.

Specifically, while a clot is frequently expressed in the Caucasian race, the frequency of its expression is relatively low in other races. Accordingly, the conventional AI model that tends to be dependent on clots has a problem in that relatively good performance is achieved only for Caucasians.

In addition, the conventional AI model that predicts a large vessel occlusion has difficulty in being applied in an environment in which accurate clinical data is not available.

### [Disclosure]

### [Technical Problem]

The present disclosure is directed to providing an AI-based apparatus and method of predicting a large vessel occlusion, which extract various characteristics from an image captured without a contrast agent and predict a large vessel occlusion based on the various characteristics.

The technical problems to be achieved by the present disclosure are not limited to the technical problems mentioned above, and other technical problems that are not mentioned will be clearly understood by those of ordinary skill in the art to which the present disclosure pertains from the description below.

### [Technical Solution]

According to an aspect of the present disclosure, an AI-based method of predicting a large vessel occlusion is disclosed. The method may include inputting a brain scan image obtained by imaging equipment, calculating a characteristic value for each brain region by masking a brain region mask onto the brain scan image and calculating a brain lesion characteristic value based on whether a brain lesion region is segmented, and inputting the characteristic value for each brain region and the brain lesion characteristic value to a prediction AI to output a confidence score for the large vessel occlusion.

According to another aspect of the present disclosure, an AI-based apparatus for predicting a large vessel occlusion is disclosed. The apparatus includes a memory storing at least one instruction and a processor that executes the at least one instruction stored in the memory based on data obtained from the memory, wherein the processor is configured to: input a brain scan image acquired by imaging equipment; calculate a characteristic value for each brain region by masking a brain region mask onto the brain scan image and calculate a brain lesion characteristic value based on whether a brain lesion region is segmented; and input the characteristic value for each brain region and the brain lesion characteristic value to a prediction AI to output a confidence score for the large vessel occlusion.

According to an aspect of the present disclosure, the brain scan image may be a CT image including an image captured in at least one tomographic layer.

According to an aspect of the present disclosure, the brain region mask may be a reference image in which a brain region is defined, the brain region being formed by merging at least one specific region of a brain into which the brain is divided.

According to an aspect of the present disclosure, the masking may be performed based on a transformation matrix obtained through a process of registering the input brain scan image to common coordinates.

According to an aspect of the present disclosure, the calculating of the characteristic value for each brain region may include filtering, based on density, pixels of a composite image that is generated by masking the brain region mask onto the brain scan image, and calculating the characteristic value for each brain region of the filtered composite image.

According to an aspect of the present disclosure, the filtering may remove, for each brain region of the composite image, a pixel whose density exceeds a threshold value from a median value of densities of the pixels.

According to an aspect of the present disclosure, the characteristic value for each brain region may be calculated based on a difference for each brain region included in the filtered composite image.

According to an aspect of the present disclosure, the characteristic value for each brain region may be calculated based on a statistical result of a difference, a ratio of average values, a ratio of standard deviations, and a ratio of volumes for each brain region, with respect to densities of the pixels for each brain region of the filtered composite image.

According to an aspect of the present disclosure, the calculating of the brain lesion characteristic value may include selecting a slice of the brain scan image that includes a cerebral artery region, among the input brain scan image, and calculating the brain lesion characteristic value from the slice.

According to an aspect of the present disclosure, the brain lesion characteristic value may be defined according to whether the brain lesion region of the slice is segmented.

According to an aspect of the present disclosure, the brain lesion region may be a region in which a clot (clot sign) that blocks a blood vessel is located.

The features briefly summarized above with respect to the present disclosure are merely exemplary aspects of the detailed description of the present disclosure described below, and do not limit the scope of the present disclosure.

### [Advantageous Effects]

According to the present disclosure, it is possible to provide an AI-based apparatus and method of predicting a large vessel occlusion, which extract various characteristics from an image captured without a contrast agent and predict a large vessel occlusion based on the various characteristics.

In addition, according to the present disclosure, the dependency of an AI on a clot can be lowered by using not only the clot but also various characteristics obtainable from an image of a scanned brain, such as an NCCT image.

In addition, according to the present disclosure, an AI model can be provided which predicts a large vessel occlusion with performance equal to or higher than a certain level even in an environment, in which accurate clinical data is not available, in consideration of a characteristic for each brain region.

In addition, according to the present disclosure, the dependency of an AI model on a clot can be reduced, and thus a performance variation across races can be reduced.

In addition, according to the present disclosure, a non-brain region can be removed more accurately by merging brain regions of an image of a scanned brain, such as an NCCT image, and filtering pixels of the brain regions.

In addition, according to the present disclosure, a loss of information depending on volume or a possibility of a false positive can be reduced by using a filtered brain scan image.

The technical effects to be achieved by the present disclosure are not limited to the technical effects mentioned above, and other technical effects that are not mentioned will be clearly understood by those of ordinary skill in the art to which the present disclosure pertains from the description below.

### [Brief Description of Drawings]

FIG. 1 is a view illustrating a server receiving a brain scan image and extracting target data.
FIG. 2 is a configuration diagram of modules constituting a server.
FIG. 3 is an overall schematic diagram of a process of predicting a large vessel occlusion.
FIG. 4 is a flowchart illustrating a process of predicting a large vessel occlusion.
FIG. 5 is a flowchart illustrating a process of calculating a characteristic value for each brain region.
FIG. 6 is a view illustrating a filtered brain scan image.
FIG. 7 is a flowchart illustrating a process of calculating a brain lesion characteristic value.
FIG. 8 is a flow diagram of a prediction AI model that predicts a large vessel occlusion based on a characteristic value for each brain region and a brain lesion characteristic value.

### [Mode for Invention]

Hereinafter, examples of the present disclosure are described in detail with reference to the accompanying drawings so that those having ordinary skill in the art may easily implement the present disclosure. However, examples of the present disclosure may be implemented in various different ways and thus the present disclosure is not limited to the examples described therein.

In describing examples of the present disclosure, well-known functions or constructions have not been described in detail since a detailed description thereof may have unnecessarily obscured the gist of the present disclosure. The same constituent elements in the drawings are denoted by the same reference numerals and a repeated or duplicative description of the same elements has been omitted.

In the present disclosure, when an element is simply referred to as being "connected to", "coupled to" or "linked to" another element, this may mean that an element is "directly connected to", "directly coupled to", or "directly linked to" another element or this may mean that an element is connected to, coupled to, or linked to another element with another element intervening therebetween. In addition, when an element "includes" or "has" another element, this means that one element may further include another element without excluding another component unless specifically stated otherwise.

In the present disclosure, the terms first, second, etc. are only used to distinguish one element from another and do not limit the order or the degree of importance between the elements unless specifically stated otherwise. Accordingly, a first element in an example may be termed a second element in another example, and, similarly, a second element in an example could be termed a first element in another example, without departing from the scope of the present disclosure.

In the present disclosure, elements are distinguished from each other for clearly describing each feature, but this does not necessarily mean that the elements are separated. In other words, a plurality of elements may be integrated in one hardware or software unit, or one element may be distributed and formed in a plurality of hardware or software units. Therefore, even if not mentioned otherwise, such integrated or distributed examples are included in the scope of the present disclosure.

For purposes of this application and the claims, using the exemplary phrase "at least one of: A; B; or C" or "at least one of A, B, or C," the phrase means "at least one A, or at least one B, or at least one C, or any combination of at least one A, at least one B, and at least one C. Further, exemplary phrases, such as "A, B, and C", "A, B, or C", "at least one of A, B, and C", "at least one of A, B, or C", etc. as used herein may mean each listed item or all possible combinations of the listed items. For example, "at least one of A or B" may refer to (1) at least one A; (2) at least one B; or (3) at least one A and at least one B.

In the present disclosure, elements described in various examples do not necessarily mean essential elements, and some of them may be optional elements. Therefore, an example composed of a subset of elements described in an example is also included in the scope of the present disclosure. In addition, examples including other elements in addition to the elements described in the various examples are also included in the scope of the present disclosure.

The advantages and features of the present disclosure and the ways of attaining them should become apparent to those of ordinary skill in the art with reference to examples of the present disclosure described below in detail in conjunction with the accompanying drawings. The examples of the present disclosure, however, may be embodied in many different forms and should not be constructed as being limited to the example examples set forth herein. Rather, the examples described herein are provided to make this disclosure more complete and to fully convey the scope of the present disclosure to those having ordinary skill in the art to which the present disclosure pertains.

Hereinafter, an AI-based apparatus and method for predicting a large vessel occlusion, which extract various characteristics from an image captured without a contrast agent and predict a large vessel occlusion based on the various characteristics, will be described with reference to the accompanying drawings.

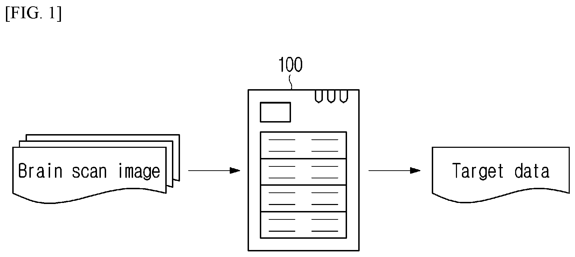

FIG. 1 is a view illustrating a server receiving a brain scan image and extracting target data.

Referring to FIG. 1, a brain scan image may be a computed tomography image captured without using a contrast agent, a fluorescent agent, or a contrast medium. As an example, the brain scan image may be an NCCT image. The brain scan image may be an image captured by dividing a brain into at least one tomographic layer, but is not limited thereto, and any image obtained by scanning a brain may be included in the present disclosure.

A server 100 may receive a brain scan image and extract target data. The target data may be a value obtained by predicting a large vessel occlusion, and as an example, a confidence score for the large vessel occlusion may be output. The confidence score may include information on a probability of being positive or negative according to a result of predicting the large vessel occlusion. In addition, the target data is not limited thereto, and may include any information that includes a degree of prediction for the large vessel occlusion.

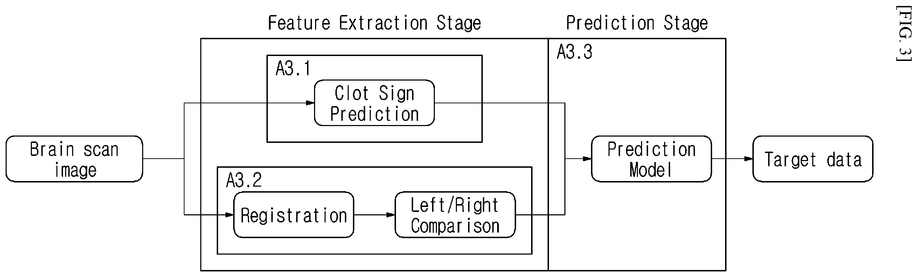

To extract the confidence score, the server 100 receives a brain scan image acquired by imaging equipment, calculates a characteristic value for each brain region by masking a brain region mask onto the brain scan image, and calculates a brain lesion characteristic value based on whether a brain lesion region is segmented. Subsequently, the server 100 inputs the characteristic value for each brain region and the brain lesion characteristic value to a prediction AI to output the confidence score for the large vessel occlusion. This will be described below in detail with reference to FIGS. 3 to 7.

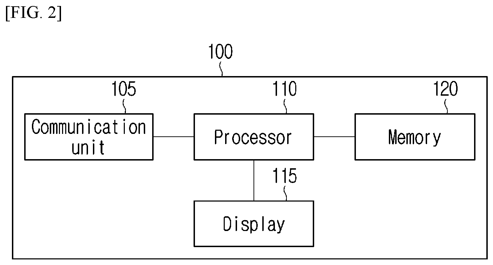

FIG. 2 is a configuration diagram of modules constituting the server.

Referring to FIG. 2, the server 100 may include a communication unit 105, a processor 110, a display 115, and a memory 120. Each constituent element is not an essential constituent element, and may have an additional configuration or may be omitted, and one configuration may be included in or combined with another configuration so that a single configuration performs a plurality of functions.

The communication unit 105 may receive a brain scan image captured by imaging equipment. As another example, in a case in which the server 100 additionally includes an imaging unit (not illustrated) capable of scanning a brain and directly generates a brain scan image, the communication unit 105 may transmit target data extracted from the brain scan image to another device. In the present disclosure, a description will be given mainly of a case in which the server 100 receives a brain scan image stored in imaging equipment or another server and extracts target data for a large vessel occlusion.

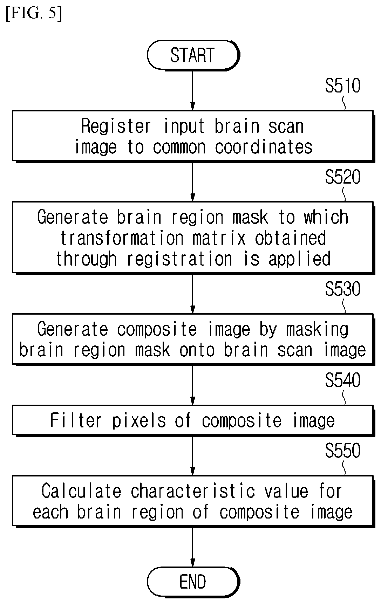

The processor 110 calculates a characteristic value for each brain region by masking a brain region mask onto the input brain scan image. The masking may be performed based on a transformation matrix obtained through a process of registering the brain scan image to common coordinates. The registration may be a process of aligning an image to a common coordinate system. This will be described below with reference to FIG. 5.

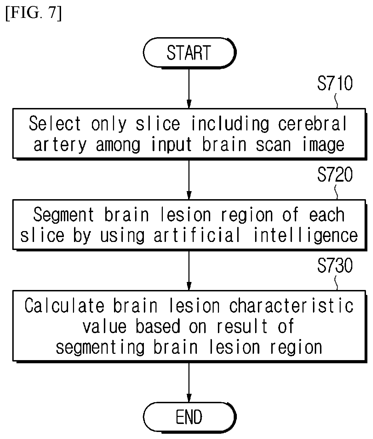

The processor 110 may calculate a brain lesion characteristic value by segmenting a brain lesion region from the input brain scan image. Specifically, the processor 110 may define a brain lesion characteristic value according to whether the brain lesion region is segmented for each slice of the brain scan image captured in at least one tomographic layer. This will be described below with reference to FIG. 7.

Subsequently, the processor 110 may output target data for a large vessel occlusion by using a prediction AI that uses the characteristic value for each brain region and the brain lesion characteristic value as inputs.

The display 115 may visualize the target data output from the processor 110 and provide the visualized target data to a user, and the memory 120 may be a non-removable memory or a removable memory and may store data required for processing the above-described processes of the processor 110 and the output data.

Subsequently, a process of extracting target data for a large vessel occlusion will be described with reference to FIG. 3.

FIG. 3 is an overall schematic diagram of a process of predicting a large vessel occlusion.

The process of predicting a large vessel occlusion may be divided into a characteristic extraction stage and a prediction stage.

Specifically, the characteristic extraction stage may include a clot sign prediction process of detecting a brain lesion region, for example, a clot (clot sign), in order to calculate the brain lesion characteristic value (A3.1 of FIG. 3). In addition, the characteristic extraction stage may include a process of performing registration of the brain scan image and comparing a left hemisphere and a right hemisphere of the brain, in order to calculate the characteristic value for each brain region (A3.2 of FIG. 3).

Specifically, the characteristic value for each brain region may be calculated based on a density of pixels or a ratio of volumes for each region of the brain, which will be described below. According to an embodiment of the present disclosure, in order to calculate the characteristic value for each brain region, the processor 110 may compare not only for the brain regions of the left and right hemispheres but also, according to another embodiment, specific regions of the brain. For example, the processor 110 may calculate the characteristic value for each brain region based on a density of pixels or a ratio of volumes for each brain region, wherein the brain region is an insula, a caudate, a putamen, an internal capsule, an anterior MCA cortex (hereinafter referred to as M1), an MCA cortex lateral to an insular ribbon (hereinafter referred to as M2), a posterior MCA cortex (hereinafter referred to as M3), a lateral MCA territory immediately superior to M2 (hereinafter referred to as M4), a lateral MCA territory immediately superior to M2 (hereinafter referred to as M5), a posterior MCA territory immediately superior to M3 (hereinafter referred to as M6), which are specific regions of the brain included in the brain scan image, or a combination of the above-described regions.

The above-described specific regions of the brain may be Alberta Stroke Program Early CT Score (ASPECTS) regions, which are regions of a scoring system that is used to measure a severity of a stroke by evaluating specific parts of the brain in an initial CT scan of a stroke patient.

The processor 110 may use a prediction AI model in the prediction stage, and may output target data by using, as inputs, a characteristic value for each brain region and a brain lesion characteristic value calculated from a brain scan image.

Hereinafter, a process of outputting target data by inputting a characteristic value for each brain region and a brain lesion characteristic value to a prediction AI will be described according to an embodiment of the present disclosure.

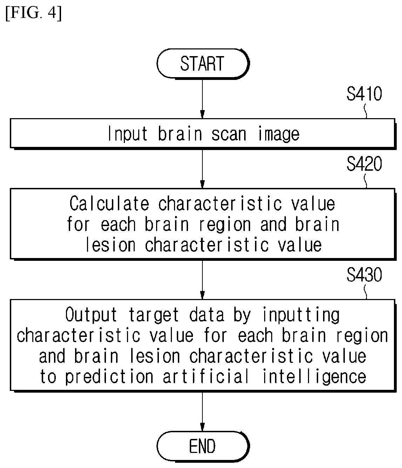

FIG. 4 is a flowchart illustrating a process of predicting a large vessel occlusion.

Referring to FIG. 4, the server 100 receives a brain scan image through the communication unit 105 (S410). According to an embodiment of the present disclosure, the brain scan image may be a computed tomography image captured by dividing a brain into at least one tomographic layer without using a contrast agent, a fluorescent agent, or a contrast medium. For example, the brain scan image may be an NCCT image.

Subsequently, the processor 110 may calculate a characteristic value for each brain region and a brain lesion characteristic value based on the input brain scan image (S420). According to an embodiment of the present disclosure, the processor 110 may calculate the characteristic value for each brain region based on a difference for each brain region of the left and right hemispheres. In addition, in order to calculate the brain lesion characteristic value, the processor 110 may detect a brain lesion region, for example, a clot (clot sign). A process of calculating the characteristic value for each brain region and the brain lesion characteristic value will be described with reference to FIGS. 5 to 7.

Next, the processor 110 may output target data by inputting the characteristic value for each brain region and the brain lesion characteristic value to a prediction AI (S430).

According to an embodiment, the target data may be a value obtained by predicting a large vessel occlusion, and as an example, a confidence score for the large vessel occlusion may be output. The confidence score may include information on a probability of being positive or negative according to a result of predicting the large vessel occlusion.

Hereinafter, a process of extracting a characteristic value for each brain region according to an embodiment of the present disclosure will be described with reference to FIG. 5.

FIG. 5 is a flowchart illustrating a process of calculating a characteristic value for each brain region.

First, the processor 110 registers the input brain scan image to common coordinates (S510). Specifically, the processor 110 may use a template image, which is a reference image serving as a reference for registration to the common coordinates. As an example, the processor 110 aligns the brain scan image, which is an image captured in at least one tomographic layer, to the common coordinates by projecting the brain scan image onto the template image.

The processor 110 may extract a transformation matrix in a process of performing the registration. The transformation matrix may be obtained in a process of aligning the brain scan image to the common coordinate system by projecting the brain scan image onto the template image, and specifically, may be a matrix for commonly transforming the brain scan image so as to geometrically match the template image.

Subsequently, the processor 110 may apply the transformation matrix obtained through the registration to a brain region mask to generate a brain region mask that geometrically matches the brain scan image aligned to the common coordinates (S520).

The brain region mask may be a reference image in which a brain region formed by merging at least one specific region of the brain is defined, and according to an embodiment, the specific region of the brain may be an ASPECTS region, which is a region of a scoring system used to measure a severity of a stroke. Accordingly, the brain region mask may be a reference image in which a total of four regions are defined, among the ASPECTS regions, the four regions being an insula region, a striatocapsular region formed by merging the caudate, the putamen, and the internal capsule, a region formed by merging M1 to M3, and a region formed by merging M4 to M6.

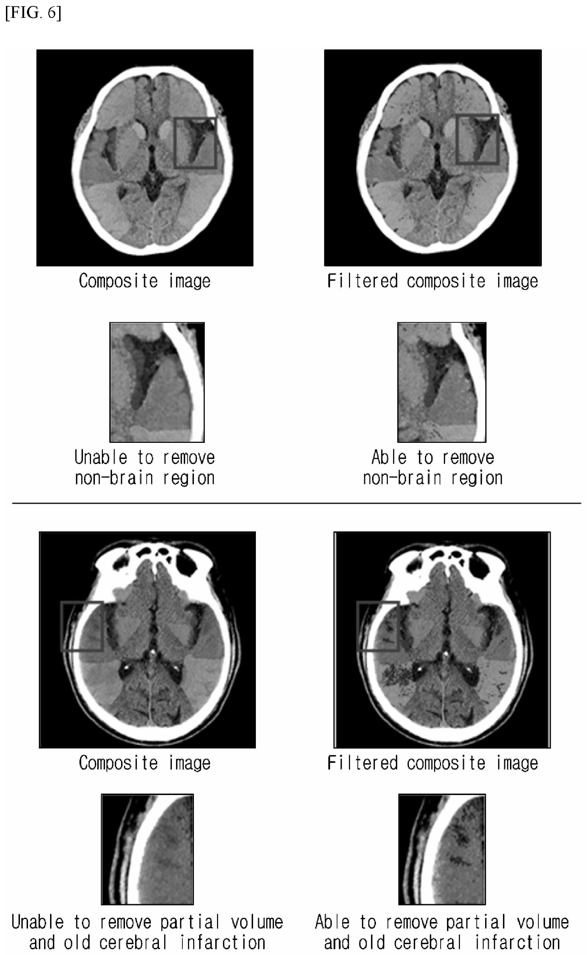

Next, the processor 110 generates a composite image by masking the brain region mask onto the brain scan image (S530). In the present disclosure, a description is given mainly of performing masking onto the brain scan image by separately generating a brain region mask aligned to the common coordinates and applying the transformation matrix to the brain region mask, but the present disclosure is not limited thereto, and the brain region mask may be masked onto the directly aligned brain scan image by using the transformation matrix, without generating a separate aligned brain region mask. The composite image thus generated will be described with reference to FIG. 6.

Subsequently, the processor 110 filters pixels of the composite image (S540). Specifically, the processor 110 removes, for each brain region of the composite image, a pixel whose density exceeds a median value of the densities of the pixels by more than a threshold value. As an example, the processor 110 may remove a pixel whose density exceeds the median value by 10 HU (Hounsfield units). The threshold value is not limited thereto, and may be set differently for each brain region.

Thus, a loss of information in terms of volume depending on a size of a pixel or a possibility of a false positive may be reduced. As an example, when a small structure captured in the brain scan image is included in one pixel, an actual size of the structure may not be accurately represented. Consequently, when a boundary of a brain region overlaps one pixel depending on a resolution, a volumetric characteristic of the brain region may become unclear. In addition, a possibility that the brain region mask is masked onto a non-brain region may be reduced, and an old cerebral infarction may be removed. This will be described with reference to FIG. 6.

FIG. 6 is a view illustrating a filtered brain scan image.

Referring to FIG. 6, according to an embodiment, when the brain region mask is masked onto the brain scan image registered to the common coordinates, four regions may be masked onto the left hemisphere and the right hemisphere of the brain. The composite image thus generated may have a masking onto a non-brain region, or may include a volumetric characteristic and an old cerebral infarction. As described with reference to FIG. 5, when a pixel whose density exceeds the median value by 10 HU is removed for each brain region, a portion masked onto a non-brain region may be removed, and an unclear volumetric characteristic (partial volume) and an old cerebral infarction may be removed.

Returning to FIG. 5 again, a characteristic value for each brain region is calculated from the filtered composite image (S550). Specifically, the characteristic value may be calculated based on a density of pixels or a ratio of volumes for each brain region included in the composite image. According to an embodiment, in order to calculate the characteristic value for each brain region, the processor 110 may compare respective brain regions of the left and right hemispheres. For example, the characteristic value for each brain region may be calculated based on a statistical result of a difference (non-equivalence score), a ratio of average values (NWU), a ratio of standard deviations (Std ratio), and a ratio of volumes for each brain region (volume), with respect to a density of pixels for each brain region of the left and right hemispheres of the composite image.

Specifically, in order to calculate the statistical result of the difference with respect to density, the processor 110 may calculate the characteristic value for each brain region by calculating, as a score between 0 and 1, a statistical result value of a difference in densities of pixels of each brain region masked onto the left and right hemisphere regions.

In addition, the processor 110 may calculate the characteristic value for each brain region by calculating a ratio of average values of densities of pixels in each brain region masked onto the left and right hemisphere regions. For example, the ratio may be calculated by an equation of 1-mean (left/right), where mean (left/right) is a ratio of an average density of pixels according to a brain region included in the left hemisphere to an average density of pixels according to a brain region included in the right hemisphere.

Likewise, the processor 110 may calculate the characteristic value for each brain region by calculating a ratio of standard deviations of densities of pixels in each brain region masked onto the left and right hemisphere regions. For example, the ratio may be calculated by an equation of 1-std (left/right), where std (left/right) is a ratio of a standard deviation of densities of pixels according to a brain region included in the left hemisphere to a standard deviation of pixels according to a brain region included in the right hemisphere.

In addition, the processor 110 may calculate the characteristic value for each brain region by calculating a ratio of volumes of each brain region masked onto the left and right hemisphere regions. For example, the ratio may be calculated by an equation of 1-vol (left/right), where vol(left/right) is a ratio of a volume of a brain region included in the left hemisphere to a volume of a brain region included in the right hemisphere.

The characteristic value for each brain region is not limited to being calculated by the above-described equations, and any method may be included as long as it calculates a statistical result of a difference (non-equivalence score), a ratio of average values (NWU), a ratio of standard deviations (Std ratio), and a ratio of volumes for each brain region (volume), with respect to a density of pixels, through comparison for each brain region of the left and right hemispheres by a certain method.

The processor 110 may take, as inputs to a prediction artificial intelligence, the respective characteristic values for the brain regions calculated by the above-described method. According to an embodiment of the present disclosure, a characteristic value for each brain region may be calculated for each of the four regions masked onto the left hemisphere and the right hemisphere of the brain, and as a result, a total of 16 characteristic values for the brain regions may be input to the prediction AI.

Subsequently, a process of calculating a brain lesion characteristic value will be described with reference to FIG. 7.

FIG. 7 is a flowchart illustrating a process of calculating a brain lesion characteristic value.

The processor 110 selects only a slice of the brain scan image that includes a cerebral artery, among the input brain scan image (S710). Specifically, the processor 110 may select only a slice that includes a middle cerebral artery (MCA) among cerebral arteries. The middle cerebral artery is one of major blood vessels of the brain, and is an artery that supplies nutrients to a lateral surface of the cerebrum.

Subsequently, the processor 110 segments a brain lesion region of each slice by using an AI (S720). According to an embodiment, the brain lesion region may be a clot (a blood clot that blocks a blood vessel). That is, the processor 110 may perform a process of detecting a clot from each slice. The processor 110 may use an AI model to detect a clot, and as an example, the processor 110 may use a convolutional neural network (CNN) structure having an encoder and decoder structure to detect a clot. For example, the processor 110 may use a U-Net. In addition, the present disclosure is not limited thereto, and the processor 110 may use any AI model that extracts a feature of an image through image segmentation.

Next, the processor 110 calculates a brain lesion characteristic value based on a result of segmenting the brain lesion region (S730). Specifically, the processor 110 may define the brain lesion characteristic value based on a result of segmenting the brain lesion region according to whether the brain lesion region of a slice of the input brain scan image is detected.

According to an embodiment, when a clot is detected in any one slice among slices of the at least one input brain scan image, the processor 110 may determine that there is a clot in the corresponding brain scan image. That is, when there is a clot in any one of the slices of the brain scan image, the processor 110 may define a brain lesion characteristic value that is determined based on the presence of the clot.

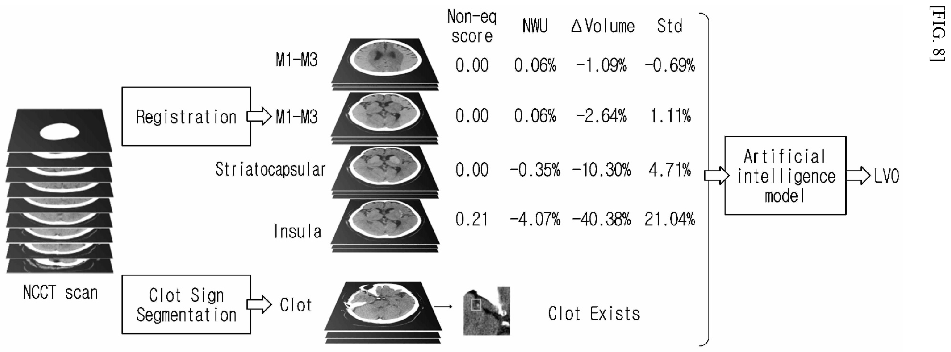

FIG. 8 is a flow diagram of a prediction AI model that predicts a large vessel occlusion based on a characteristic value for each brain region and a brain lesion characteristic value.

A characteristic value for each brain region and a brain lesion characteristic value extracted by the above-described process are used as input values of a prediction AI model. According to an embodiment of the present disclosure, a characteristic value for each brain region is calculated for each of the four regions masked onto the brain scan image, so that a total of 16 characteristic values for the brain regions may be input to the prediction AI model. For example, a characteristic value for each brain region calculated based on a ratio of average values, a ratio of standard deviations, and a ratio of volumes may be input as a percentage, and a characteristic value for each brain region based on a statistical result of a difference in densities of pixels may be input as a value between 0 and 1.

Subsequently, the processor 110 inputs the characteristic value for each brain region and the brain lesion characteristic value to the prediction AI to output a confidence score for the large vessel occlusion.

Specifically, according to an embodiment of the present disclosure, the processor 110 may output a confidence score for the large vessel occlusion, which is a value obtained by predicting the large vessel occlusion, and the confidence score may include information on a probability of being positive or negative according to a result of predicting the large vessel occlusion.

Although the above-described exemplary methods of the present disclosure are represented as a series of operations for clarity of description, this is not intended to limit the order in which the steps are performed, and the steps may be performed concurrently or in a different order, if necessary. To implement the method according to the present disclosure, other steps may be included in addition to the illustrated steps, some of the steps may be excluded while the remaining steps are included, or some of the steps may be excluded while additional other steps are included.

The various embodiments of the present disclosure are not intended to list all possible combinations but to describe representative aspects of the present disclosure, and the matters described in the various embodiments may be applied independently or in combination of two or more.

In addition, various examples of the present disclosure may be implemented in hardware, firmware, software, or a combination thereof. In the case of implementing the present disclosure by hardware, the present disclosure can be implemented with application specific integrated circuits (ASICs), Digital signal processors (DSPs), digital signal processing devices (DSPDs), programmable logic devices (PLDs), field programmable gate arrays (FPGAs), general processors, controllers, microcontrollers, microprocessors, etc.

The scope of the disclosure includes software or machine-executable commands (e.g., an operating system, an application, firmware, a program, etc.) for enabling operations according to the methods of various examples to be executed on an apparatus or a computer, a non-transitory computer-readable medium having such software or commands stored thereon and executable on the apparatus or the computer.

### [Industrial Applicability]

The present invention may be used for an AI-based apparatus for predicting a large vessel occlusion, which extracts various characteristics from an image captured without a contrast agent and predicts a large vessel occlusion based on the various characteristics.

## Claims

1. An artificial intelligence-based method of predicting a large vessel occlusion, the method comprising:
inputting a brain scan image obtained by imaging equipment;
calculating a characteristic value for each brain region by masking a brain region mask onto the brain scan image, and calculating a brain lesion characteristic value based on whether a brain lesion region is segmented; and
outputting a confidence score for the large vessel occlusion by inputting the characteristic value for each brain region and the brain lesion characteristic value to a prediction artificial intelligence.

2. The method of Claim 1, wherein the brain scan image is a computed tomography image including an image captured in at least one tomographic layer.

3. The method of Claim 1, wherein the brain region mask is a reference image in which a brain region is defined and formed by merging at least one specific region of a brain into which the brain is divided.

4. The method of Claim 1, wherein the masking is performed based on a transformation matrix obtained through a process of registering the input brain scan image to common coordinates.

5. The method of Claim 1, wherein the calculating of the characteristic value for each brain region comprises:
filtering, based on density, pixels of a composite image generated by masking the brain region mask onto the brain scan image; and
calculating the characteristic value for each brain region of the filtered composite image.

6. The method of Claim 5, wherein the filtering removes, for each brain region of the composite image, a pixel whose density exceeds a median value of densities of the pixels by more than a threshold value.

7. The method of Claim 5, wherein the characteristic value for each brain region is calculated based on a difference for each brain region included in the filtered composite image.

8. The method of Claim 7, wherein the characteristic value for each brain region is calculated based on a statistical result of a difference, a ratio of average values, a ratio of standard deviations, and a ratio of volumes for each brain region, with respect to densities of the pixels for each brain region of the filtered composite image.

9. The method of Claim 2, wherein the calculating of the brain lesion characteristic value comprises:
selecting a slice of the brain scan image that includes a cerebral artery region, among the input brain scan image; and
calculating the brain lesion characteristic value from the slice.

10. The method of Claim 9, wherein the brain lesion characteristic value is defined according to whether the brain lesion region of the slice is segmented.

11. The method of Claim 10, wherein the brain lesion region is a region in which a clot (clot sign) that blocks a blood vessel is located.

12. An artificial intelligence-based apparatus for predicting a large vessel occlusion, comprising:
a memory storing at least one instruction; and
a processor that executes the at least one instruction stored in the memory based on data obtained from the memory,
wherein the processor is configured to:
input a brain scan image acquired by imaging equipment,
calculate a characteristic value for each brain region by masking a brain region mask onto the brain scan image, and calculate a brain lesion characteristic value based on whether a brain lesion region is segmented, and
output a confidence score for the large vessel occlusion by inputting the characteristic value for each brain region and the brain lesion characteristic value to a prediction artificial intelligence.

13. The apparatus of Claim 12, wherein the brain scan image is a computed tomography image including an image captured in at least one tomographic layer.

14. The apparatus of Claim 12, wherein the brain region mask is a reference image in which a brain region is defined and formed by merging at least one specific region of a brain into which the brain is divided.

15. The apparatus of Claim 12, wherein the masking is performed based on a transformation matrix obtained through a process of registering the input brain scan image to common coordinates.

16. The apparatus of Claim 12, wherein the processor is configured to:
filter, based on density, pixels of a composite image generated by masking the brain region mask onto the brain scan image, and
calculate the characteristic value for each brain region of the filtered composite image.

17. The apparatus of Claim 16, wherein the filtering removes, for each brain region of the composite image, a pixel whose density exceeds a median value of densities of the pixels by more than a threshold value.

18. The apparatus of Claim 16, wherein the characteristic value for each brain region is calculated based on a difference for each brain region included in the filtered composite image.

19. The apparatus of Claim 18, wherein the characteristic value for each brain region is calculated based on a statistical result of a difference, a ratio of average values, a ratio of standard deviations, and a ratio of volumes for each brain region, with respect to densities of the pixels for each brain region of the filtered composite image.

20. The apparatus of Claim 13, wherein the processor is configured to:
select a slice of the brain scan image that includes a cerebral artery region, among the input brain scan image, and
calculate a brain lesion characteristic value from the slice.

21. The apparatus of Claim 20, wherein the brain lesion characteristic value is defined according to whether a brain lesion region of the slice is segmented.

22. The apparatus of Claim 21, wherein the brain lesion region is a region in which a clot (clot sign) that blocks a blood vessel is located.
